# EUROPEAN PATENT SPECIFICATION

(11) **EP 3 607 036 B1**
(45) Date of publication and mention of the grant of the patent: **07.02.2024**
(21) Application number: 18707050.3
(22) Date of filing: 01.03.2018
(51) Int. Cl.: C11D 3/386

(54) **DETERGENT COMPOSITIONS COMPRISING BACTERIAL MANNANASES**
WASCHMITTELZUSAMMENSETZUNGEN MIT BAKTERIELLEN MANNANASEN
COMPOSITIONS DÉTERGENTES COMPRENANT DES MANNANASES BACTÉRIENNES

(30) Priority: 05.04.2017 EP 17164901
(43) Date of publication of application: 12.02.2020
(73) Proprietor: AB Enzymes GmbH, 64293 Darmstadt (DE)
(72) Inventor: HERBST, Daniela, 40237 Düsseldorf (DE); WIELAND, Susanne, 41541 Zons/Dormagen (DE); MUSSMANN, Nina, 47877 Willich (DE); LEINONEN, Taija, 11100 Riihimäki (FI); VALTAKARI, Leena, 05200 Rajamäki (FI); RACHINGER, Michael, 63762 Großostheim (DE); JUNTUNEN, Kari, 02680 Espoo (FI); DOLLAK, Daniela, 64589 Stockstadt a. Rhein (DE); LORENZ, Patrick, 64653 Lorsch (DE); VEHMAANPERÄ, Jari, 00980 Helsinki (FI); OJAPALO, Pentti, 04360 Tuusula (FI); PURANEN, Terhi, 01900 Nurmijärvi (FI); JÄRVINEN, Kristiina, 02940 Espoo (FI)
(74) Representative: Kraus & Lederer Patentanwälte PartGmbB
(86) International application number: PCT/EP2018/055007
(87) International publication number: WO 2018/184767

(56) References cited:
- WO-A2-99/64619
- DHAWAN SAMRITIL ET AL: "Microbial mannanases: An overview of production and applications", CRC CRITICAL REVIEWS IN BIOTECHNO, CRC PRESS, BOCA RATON, FL, US, vol. 27, no. 4, 1 January 2007 (2007-01-01), pages 197-216, XP009161462, ISSN: 0738-8551, DOI: 10.1080/07388550701775919

## Description

### FIELD OF THE INVENTION

This invention relates to novel detergent compositions comprising bacterial mannanase enzymes. The detergent compositions comprising bacterial mannanases are useful in laundry and cleaning applications wherein degradation or modification of mannan is desired. The invention also relates to the use of said detergent compositions in laundry and cleaning applications as well as a method for degrading mannan.

### BACKGROUND

Mannans are mannose containing polysaccharides found in various plants. Mannans are poorly soluble in an aqueous environment and their physicochemical properties give rise to viscous dispersions. Additionally, mannans have high water- binding capacity. All of these characteristics cause problems in several industries including brewing, baking, animal nutrition, and laundry and cleaning applications.

In plant based diets different β-mannans are present and depending on their amounts and properties they can compromise nutrient digestion, microbial colonisation and growth performance. Enzymatic degradation of mannans reduces digesta viscosity of high water soluble mannans and leads to production of manno-oligosaccharides that may form water-insoluble linear mannans present in leguminoseae. Mannanase increases average daily gain, feed efficiency, weight uniformity and livability in all monogastric animals.

For animal feed applications, such as feed for monogastric animals with cereal diets, mannan is a contributing factor to viscosity of gut contents and it thereby adversely affects the feed digestibility and animal growth rate. For ruminants, mannan represents a substantial component of fiber intake and a more complete digestion of mannan would facilitate higher feed conversion efficiencies.

For laundry and cleaning applications detergent compositions comprising mannanase can be used to degrade mannan. However, providing mannanases that are stable in varying storage and use conditions while still showing good mannan degrading activity is difficult.

It is an object of the present invention to provide detergent compositions comprising novel enzymes exhibiting mannanase activity when applied in these detergent compositions. It is a further object of the present invention to provide detergent compositions having increased stain removal performance on mannan containing stains.

WO 99/646169 discloses a detergent composition for removing stains on textile comprising mannanase enzymes having various sequences.

Dhawan Samritil et al. provides an overview on microbial mannanases in particular on the complex mannan structure and the microbial enzyme complex involved in its complete breakdown mannanase sources, protection conditions and the applications in the commercial sector.

### SUMMARY

According to the first aspect of the invention there is provided a detergent composition comprising at least one enzyme having an amino acid sequence having at least 93% sequence identity to the amino acid sequence of SEQ ID NO: 12 (Man6), wherein the at least one enzyme has mannan degrading activity.

In one embodiment of the invention the at least one enzyme has an amino acid sequence having at least 94%, at least 95%, at least 96%, at least 97%, at least 98%, or at least 99% sequence identity to SEQ ID NO: 12.

The mannanases comprised in the detergent composition of the invention are suitable for degrading and modifying mannan containing material in various chemical environments, preferably in detergent compositions.

In one embodiment of the present invention the detergent composition further comprises one or more additional enzymes selected from the group consisting of protease, lipase, cutinase, amylase, carbohydrase, cellulase, pectinase, pectatlyase, mannanase, arabinase, galactanase, xylanase, oxidase, xanthanase, laccase, and/or peroxidase, preferably selected from the group consisting of proteases, amylases, cellulases and lipases.

In a further embodiment of the present invention the detergent composition is in form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition.

The present invention furthermore relates to the use of the detergent composition as herein disclosed for degrading mannan.

In a further embodiment the present invention relates to the use of the detergent composition as herein disclosed in a laundry process.

The present invention furthermore relates to a method for removing a stain from a surface, comprising contacting the surface with a detergent composition as herein disclosed.

The present invention also relates to a method for degrading mannan comprising applying a detergent composition as herein disclosed to mannan, preferably wherein the mannan is on the surface of a textile.

### BRIEF DESCRIPTION OF THE FIGURES

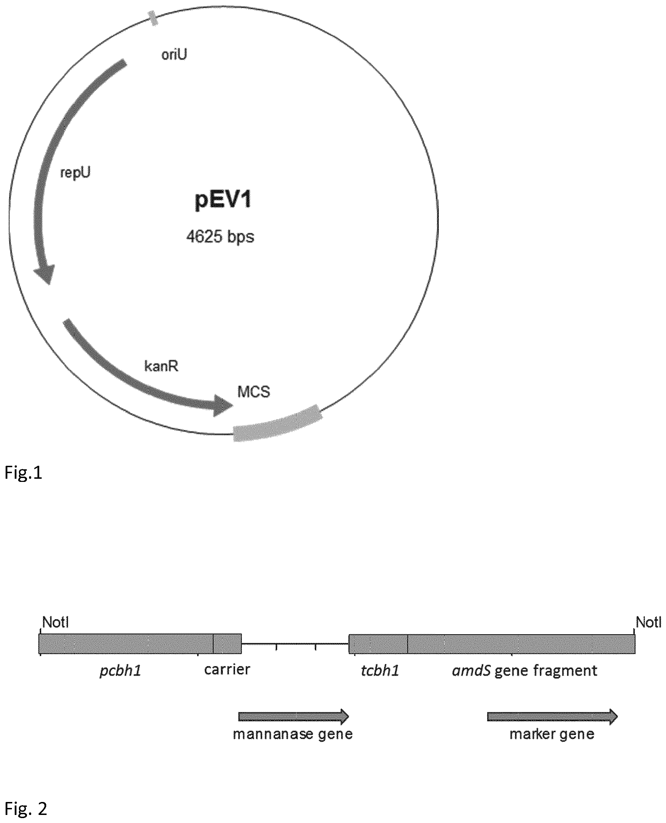

**Fig 1** shows schematic representation of vector pEV1 for replication in *Bacillus*
**Fig 2** schematically shows the expression cassettes used in the transformation of *Trichoderma reesei* protoplasts for overproducing the recombinant mannanase proteins (Man6 (invention), Man7 (referential) and Man14 (referential)). The mannanase genes were under the control of *T. reesei cel7A*/*cbh1* promoter (p*cbh1*) and the termination of the transcription was ensured by using *T. reesei cel7A*/*cbh1* terminator sequence (t*cbh1*)*.* The *amd*S gene was included as a transformation marker.

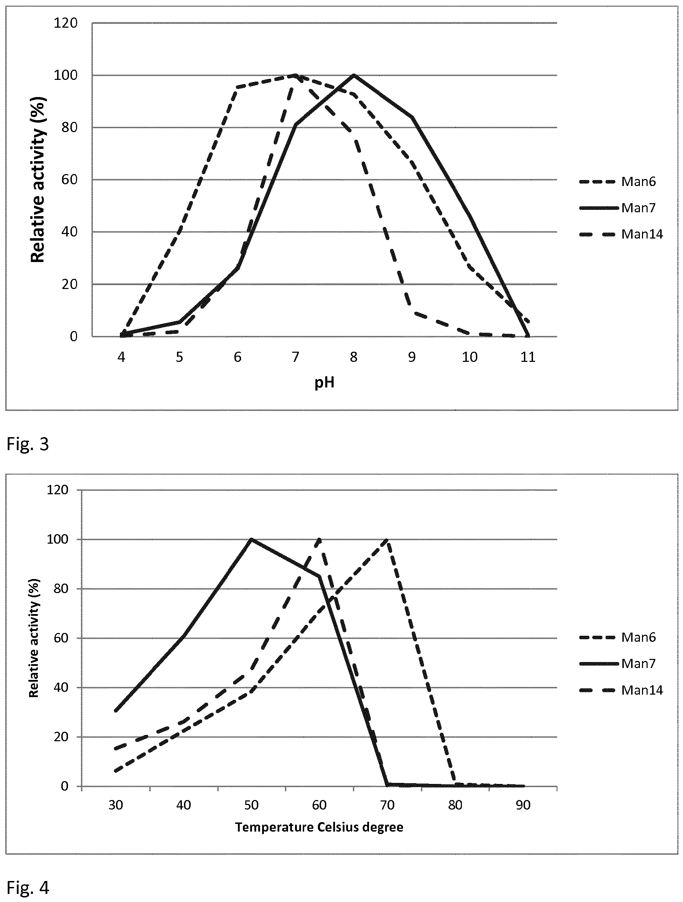

**Fig 3** shows the temperature profile of recombinant Man6 (invention), Man7 (referential) and Man14 (referential) (Bacillus produced) mannanase assayed in 40 mM Britton-Robinson buffer pH 7 using 10 min reaction time, Azurine-crosslinked carob galactomannan was used as a substrate. All measurements were made at least duplicates. The data points are averages of separate measurements.
**Fig 4** describes the effect of pH on the activity of recombinant Man6 (invention), Man7 (referential) and Man14 (referential) (Bacillus produced) mannanase protein in 40 mM Britton-Robinson buffer at pH 4 to pH 11. Reaction temperature was 50°C and the reaction time was 10 min. Azurine-crosslinked carob galactomannan was used as a substrate. All measurements were made at least duplicates. The data points are averages of separate measurements.

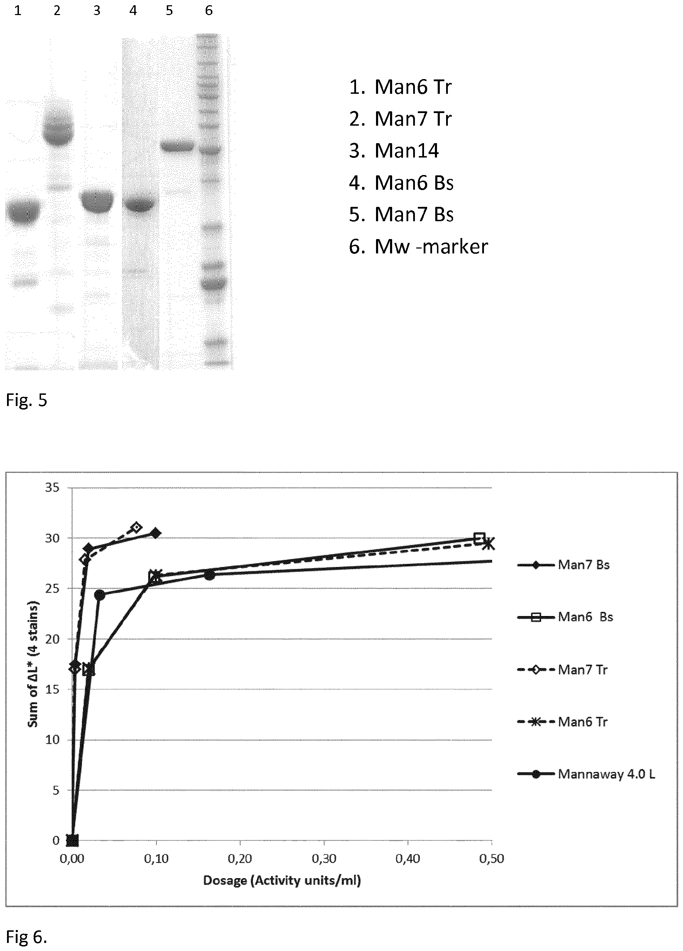

**Fig 5** shows SDS PAGE analysis of bacterial mannanases.
**Fig 6** describes the stain removal performance of Man 6 (invention) and Man7 (referential) (produced in *Bacillus* and *Trichoderma*) as an increase of lightness (sum of ΔL*of 4 stains) in the presence of 4,4 g/l of Commercial heavy duty liquid detergent A at 40°C,16 °dH, 60 min, pH approx. 8.3 and enzymes dosed as activity units. Commercial preparation Mannaway^{®} 4,0 L was used for comparison.

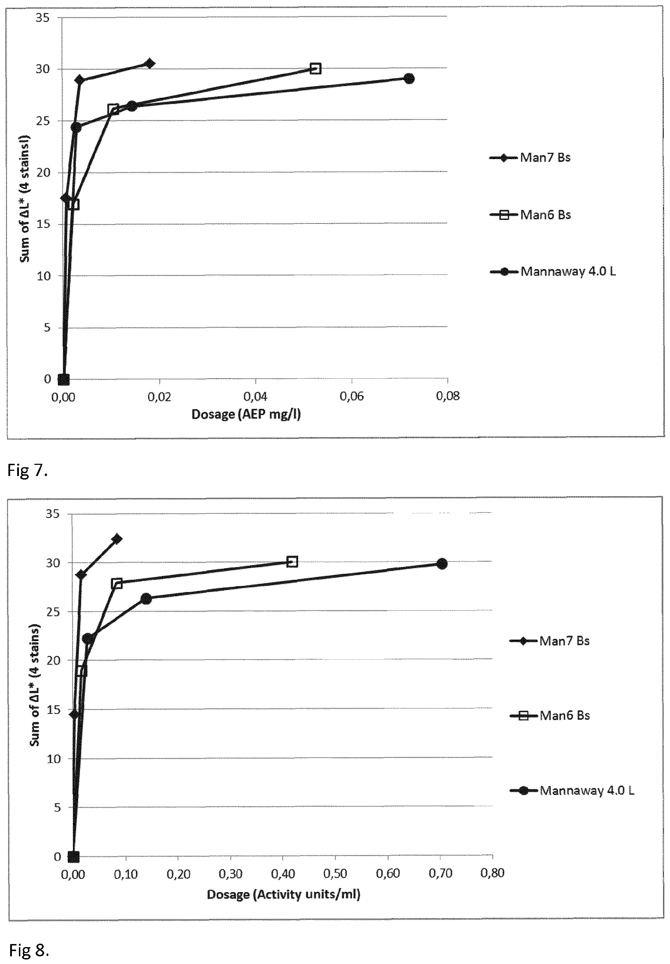

**Fig 7** describes the stain removal performance of Man 6 (invention) and Man7 (referential) (produced in *Bacillus*) as an increase of lightness (sum of ΔL*of 4 stains) in the presence of 4.4 g/l of Commercial heavy duty liquid detergent A at 40°C, 16 °dH, 60 min, pH approx. 8.3 and enzymes dosed as active enzyme protein (AEP). Commercial preparation Mannaway^{®} 4,0 L was used for comparison.
**Fig 8** describes the stain removal performance of Man 6 (invention) and Man7 (referential) (produced in *Bacillus*) as an increase of lightness (sum of ΔL*of 4 stains) in the presence of 3.8 g/l of Commercial color detergent powder at 40°C, 16 °dH, 60 min, pH approx. 10. and enzymes dosed as activity units. Commercial preparation Mannaway^{®} 4,0 L was used for comparison.

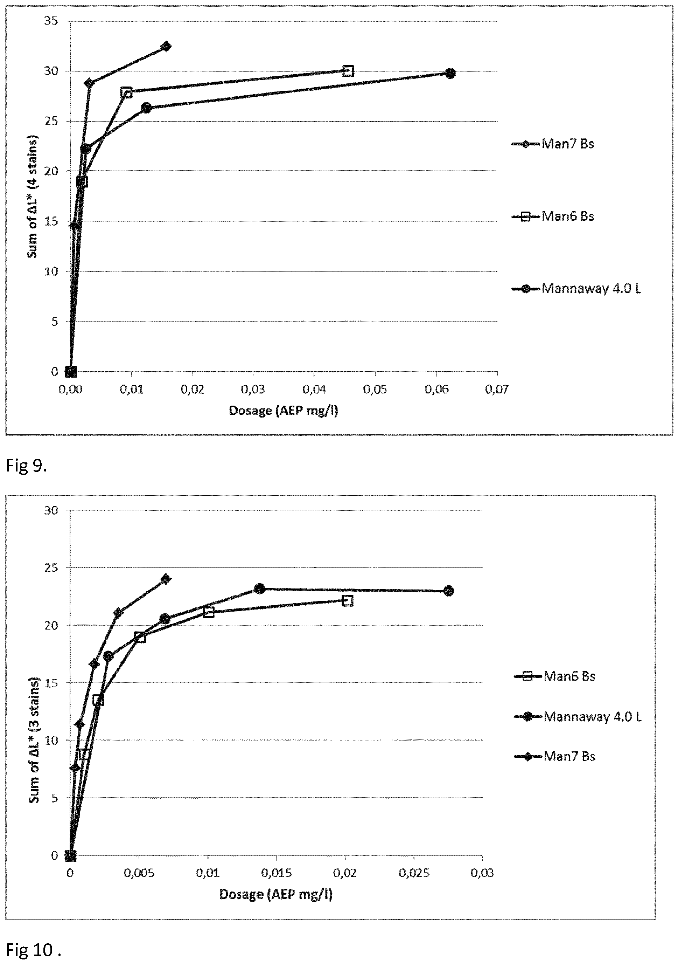

**Fig 9** describes the stain removal performance of Man 6 (invention) and Man7 (referential) (produced in *Bacillus*) as an increase of lightness (sum of ΔL*of 4 stains) in the presence of 3.8 g/l of Commercial color detergent powder at 40°C, 16 °dH, 60 min, pH approx. 10 and enzymes dosed as active enzyme protein. Commercial preparation Mannaway^{®} 4,0 L was used for comparison.
**Fig 10** describes the stain removal performance of Man 6 (invention) and Man7 (referential) (produced in *Bacillus*) as an increase of lightness (sum of ΔL* of 3 stains) in the presence of 4,2 g/l of Commercial bleach detergent powder at 40°C, 16 °dH, 60 min, pH approx. 9.5 and enzymes dosed as active enzyme protein. Commercial preparation Mannaway^{®} 4,0 L was used for comparison.

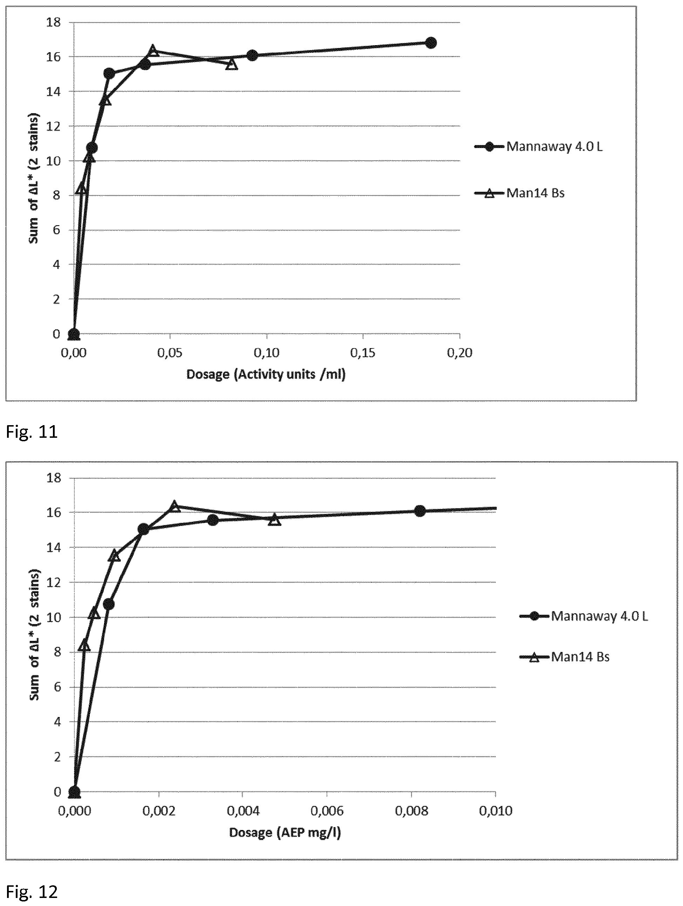

**Fig 11** the stain removal performance of Man14 (referential) (produced in *Bacillus*) as an increase of lightness (sum of ΔL*of 2 stains) in the presence of 5 g/l of Commercial heavy duty liquid detergent B at 40°C, 16 °dH, 60 min, pH approx. 8.3 and enzymes dosed as activity units. Commercial preparation Mannaway^{®} 4,0 L was used for comparison.
**Fig 12** the stain removal performance of Man14 (referential) (produced in *Bacillus*) as an increase of lightness (sum of ΔL*of 2 stains) in the presence of 5 g/l of Commercial heavy duty liquid detergent B at 40°C, 16 °dH, 60 min, pH approx. 8.3 and enzymes dosed as active enzyme protein. Commercial preparation Mannaway^{®} 4,0 L was used for comparison.

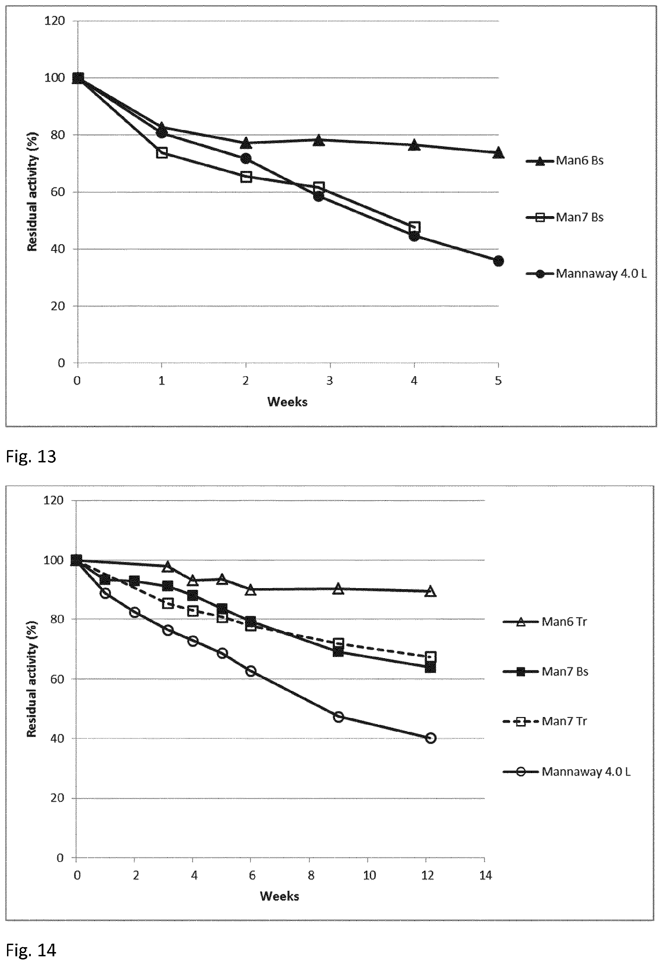

**Fig 13** describes the stability of Man 6 and Man7 (referential) (produced in *Bacillus*) in liquid detergent (OMO Color) at 37°C. Commercial preparation Mannaway^{®} 4,0 L was used for comparison
**Fig 14** describes the stability of Man 6 (produced in *Bacillus*) in Commercial heavy duty liquid detergent A. Commercial preparation Mannaway^{®} 4,0 L was used for comparison.

### SEQUENCE LISTINGS

SEQ ID NO: 1 Sequence of the oligonucleotide primer Man6_1
SEQ ID NO: 2 Sequence of the oligonucleotide primer Man6_2
SEQ ID NO: 3 Sequence of the oligonucleotide primer Man7_1 (referential)
SEQ ID NO: 4 Sequence of the oligonucleotide primer Man7_2 (referential)
SEQ ID NO: 5 Sequence of the oligonucleotide primer Man14_1 (referential)
SEQ ID NO: 6 Sequence of the oligonucleotide primer Man14_2 (referential)
SEQ ID NO: 7 Sequence of the oligonucleotide primer Vec_1
SEQ ID NO: 8 Sequence of the oligonucleotide primer Vec_2
SEQ ID NO: 9 The nucleotide sequence of the *Bacillus clausii man6*
SEQ ID NO: 10 The nucleotide sequence of the *Bacillus clausii man6* without signal peptide encoding sequence and with codon optimization to *Trichoderma reesei*
SEQ ID NO: 11 The deduced amino acid sequence of the *Bacillus clausii* Man6
SEQ ID NO: 12 The deduced amino acid sequence of the *Bacillus clausii* Man6 without signal peptide
SEQ ID NO: 13 The nucleotide sequence of the *Bacillus hemicellulosilyticus man7* (referential)
SEQ ID NO:14 The nucleotide sequence of the *Bacillus hemicellulosilyticus man7* without signal peptide encoding sequence and with codon optimization to *Trichoderma reesei* (referential)
SEQ ID NO: 15 The deduced amino acid sequence of the *Bacillus hemicellulosilyticus* Man7 (referential)
SEQ ID NO: 16 The deduced amino acid sequence of the *Bacillus hemicellulosilyticus* Man7 without signal peptide (referential)
SEQ ID NO: 17 The nucleotide sequence of the *Virgibacillus soli man14* (referential)
SEQ ID NO: 18 The nucleotide sequence of the *Virgibacillus soli man14* without signal peptide encoding sequence and with codon optimization to *Trichoderma reesei* (referential)
SEQ ID NO: 19 The deduced amino acid sequence of the *Virgibacillus soli* Man14 (referential)
SEQ ID NO: 20 The deduced amino acid sequence of the *Virgibacillus soli* Man14 without signal peptide (referential)
SEQ ID NO: 21 Sequence of the oligonucleotide primer BMAN1
SEQ ID NO: 22 Sequence of the oligonucleotide primer BMAN2
SEQ ID NO: 23 Sequence of the oligonucleotide primer BMAN3
SEQ ID NO: 24 Sequence of the oligonucleotide primer BMAN4
SEQ ID NO: 25 The nucleotide sequence of *Bacillus pumilus man31* (referential)
SEQ ID NO: 26 The deduced amino acid sequence of the *Bacillus pumilus* Man31 (referential)
SEQ ID NO: 27 The nucleotide sequence of the *Bacillus amyloliquefaciens man32* (referential)
SEQ ID NO: 28 The deduced amino acid sequence of the *Bacillus amyloliquefaciens* Man32 (referential)
SEQ ID NO: 29 The nucleotide sequence of the *Amphibacillus xylanus man33* (referential)
SEQ ID NO: 30 The deduced amino acid sequence of the *Amphibacillus xylans* Man33 (referential)
SEQ ID NO: 31 The nucleotide sequence of the *Paenibacillus polymyxa man34* (referential)
SEQ ID NO: 32 The deduced amino acid sequence of the *Paenibacillus polymyxa* Man34 (referential)
SEQ ID NO: 33 The nucleotide sequence of the *Bacillus hemicellulosilyticus man35* (referential)
SEQ ID NO: 34 The deduced amino acid sequence of the *Bacillus hemicellulosilyticus* Man35 (referential)
SEQ ID NO: 35 The nucleotide sequence of the *Bacillus alcalophilus man36* (referential)
SEQ ID NO: 36 The deduced amino acid sequence of the *Bacillus alcalophilus* Man36 (referential)
SEQ ID NO: 37 The nucleotide sequence of the *Bacillus* sp. *man37* (referential)
SEQ ID NO: 38 The deduced amino acid sequence of the *Bacillus* sp. Man37 (referential)
SEQ ID NO: 39 The nucleotide sequence of the *Bacillus circulans man38* (referential)
SEQ ID NO: 40 The deduced amino acid sequence of the *Bacillus circulans* Man38 (referential)
SEQ ID NO: 41 The nucleotide sequence of the *Paenibacillus* sp. *man39* (referential)
SEQ ID NO: 42 The deduced amino acid sequence of the *Paenibacillus* sp. Man39 (referential)
SEQ ID NO: 43 The nucleotide sequence of the *Bacillus circulans man40* (referential)
SEQ ID NO: 44 The deduced amino acid sequence of the *Bacillus circulans* Man40 (referential)
SEQ ID NO: 45 The nucleotide sequence of the *Bacillus nealsonii man41* (referential)
SEQ ID NO: 46 The deduced amino acid sequence of the *Bacillus nealsonii* Man41 (referential)
SEQ ID NO: 47 The nucleotide sequence of the *Bacillus circulans man42* (referential)
SEQ ID NO: 48 The nucleotide sequence of the *Bacillus circulans* Man42 (referential)

### DETAILED DESCRIPTION

As used herein, "isolated" means a substance in a form or environment that does not occur in nature. Non-limiting examples of isolated substances include (1) any non-naturally occurring substance, (2) any substance including any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing or decreasing the amount of the substance relative to other components with which it is naturally associated (e.g., recombinant production in a host cell; one or multiple copies of a gene encoding the substance; and use of an alternative promoter to the promoter naturally associated with the gene encoding the substance).In an embodiment a polypeptide, enzyme, polynucleotide, host cell or composition of the invention is isolated.

As used herein, the term "comprising" includes the broader meanings of "including", "containing", and "comprehending", as well as the narrower expressions "consisting of" and "consisting only of".

As used herein, "fragment" means a protein or a polynucleotide having one or more amino acids or nucleotides deleted. In the context of DNA, a fragment includes both single stranded and double stranded DNA of any length. A fragment may be an active fragment which has the biological function, such as enzyme activity or regulatory activity, of the protein or the polynucleotide. A fragment may also be an inactive fragment, i.e. it does not have one or more biological effects of the native protein or polynucleotide.

As used herein, "variant" means a fragment of sequence (nucleotide or amino acid) inserted or deleted by one or more nucleotides/amino acids or which is chemically modified.

As used herein, a "peptide" and a "polypeptide" are amino acid sequences including a plurality of consecutive polymerized amino acid residues. For purpose of this invention, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

The term "polynucleotide" denotes a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules.

As used herein, "modification", "modified", and similar terms in the context of polynucleotides refer to modification in a coding or a non-coding region of the polynucleotide, such as a regulatory sequence, 5' untranslated region, 3' untranslated region, up-regulating genetic element, down-regulating genetic element, enhancer, suppressor, promoter, exon, or intron region. The modification may in some embodiments be only structural, having no effect on the biological effect, action or function of the polynucleotide. In other embodiments the modification is a structural modification which provides a change in the biological effect, action or function of the polynucleotide. Such a modification may enhance, suppress or change the biological function of the polynucleotide.

As used herein, "identity" means the percentage of exact matches of amino acid residues between two aligned sequences over the number of positions where there are residues present in both sequences. When one sequence has a residue with no corresponding residue in the other sequence, the alignment program allows a gap in the alignment, and that position is not counted in the denominator of the identity calculation. Identity is a value determined with the Pairwise Sequence Alignment tool EMBOSS Needle at the EMBL-EBI website (www.ebi.ac.uk/Tools/psa/emboss_needle/).

As used herein, "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Non-limiting examples of a host cell are fungal cells, filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora and Humicola*; more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium*; more preferably from the group consisting of *Trichoderma reesei (Hypocrea jecorina), T. citrinoviridae, T. longibrachiatum, T. virens, T. harzianum, T. asperellum, T. atroviridae, T. parareesei,* , *Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,* most preferably *Trichoderma reesei.* Non-limiting examples of a host cell are bacterial cells, preferably gram positive Bacilli (e.g. *B*. *subtilis, B. licheniformis, B. megaterium, B. amyloliquefaciens, B. pumilus*)*,* actinomycetales (e.g. *Streptomyces* sp.) and yeasts (e.g. *Saccharomyces cerevisiae, Pichia pastoris, Yarrowia lipolytica*)

In an example embodiment of the host cell is a fungal cell, preferably a filamentous fungal cell, such as *Trichoderma* or *Trichoderma reesei.* In an example embodiment of the host cell is a bacterial cell, preferably a gram positive *Bacillus* cell, such as *B*. *subtilis, B. licheniformis, B. megaterium, B. amyloliquefaciens, B. pumilus.*

A "recombinant cell" or "recombinant host cell" refers to a cell or host cell that has been genetically modified or altered to comprise a nucleic acid sequence which is not native to said cell or host cell. In an embodiment the genetical modification comprises integrating the polynucleotide in the genome of the host cell. In another embodiment the polynucleotide is exogenous compared to the host cell.

As used herein, "expression" includes any step involved in the production of a polypeptide in a host cell including, but not limited to, transcription, translation, post-translational modification, and secretion. Expression may be followed by the harvesting, i.e. recovering, the host cells or the expressed product.

The term "expression vector" denotes a DNA molecule, linear or circular, that comprises a segment encoding a polypeptide of interest operably linked to additional segments that provide for its transcription. Such additional segments may include promoter and terminator sequences, and may optionally include one or more origins of replication, one or more selectable markers, an enhancer, a polyadenylation signal, carrier and the like. Expression vectors are generally derived from plasmid or viral DNA, or may contain elements of both. The expression vector may be any expression vector that is conveniently subjected to recombinant DNA procedures, and the choice of vector will often depend on the host cell into which the vector is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The term "recombinant expressed" or "recombinantly expressed" used herein in connection with expression of a polypeptide or protein is defined according to the standard definition in the art.

The term "obtained from" and "obtainable" as used herein in connection with a specific microbial source, means that the polynucleotide and/or polypeptide is produced by the specific source (homologous expression), or by a cell in which a gene from the source has been inserted (heterologous expression).

The term "enzyme composition" means either a conventional enzymatic fermentation product, possibly isolated and purified, from a single species of a microorganism, such preparation usually comprising a number of different enzymatic activities; or a mixture of monocomponent enzymes, preferably enzymes derived from bacterial or fungal species by using conventional recombinant techniques, which enzymes have been fermented and possibly isolated and purified separately and which may originate from different species, preferably fungal or bacterial species or the fermentation product of a microorganism which acts as a host cell for expression of a recombinant mannanase, but which microorganism simultaneously produces other enzymes.

The term "operably linked", when referring to DNA segments, denotes that the segments are arranged so that they function in concert for their intended purposes, e.g. transcription initiates in the promoter and proceeds through the coding segment to the terminator

The term "promoter" denotes a portion of a gene containing DNA sequences that provide for the binding of RNA polymerase and initiation of transcription. Promoter sequences are commonly, but not always, found in the 5' non-coding regions of genes.

The term "secretory signal sequence" denotes a DNA sequence that encodes a polypeptide (a "secretory peptide") that, as a component of a larger polypeptide, directs the larger polypeptide through a secretory pathway of a host cell in which it is synthesized. The secretory signal sequence can be native or it can be replaced with secretory signal sequence or carrier sequence from another source. Depending on the host cell, the larger peptide may be cleaved to remove the secretory peptide during transit through the secretory pathway.

The term "core region" denotes a domain of an enzyme which may or may not have been modified or altered, but which has retained its original activity; the catalytic domain as known in the art has remained functional.

By the term "linker" or "spacer" is meant a polypeptide comprising at least two amino acids which may be present between the domains of a multidomain protein, for example an enzyme comprising an enzyme core and a binding domain such as a carbohydrate binding module (CBM) or any other enzyme hybrid, or between two proteins or polypeptides expressed as a fusion polypeptide, for example a fusion protein comprising two core enzymes. For example, the fusion protein of an enzyme core with a CBM is provided by fusing a DNA sequence encoding the enzyme core, a DNA sequence encoding the linker and a DNA sequence encoding the CBM sequentially into one open reading frame and expressing this construct.

The term "detergent composition", includes unless otherwise indicated, granular or powder-form all-purpose or heavy-duty washing agents, especially cleaning detergents; liquid, gel or paste-form all-purpose washing agents, especially the so- called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners; metal cleaners; as well as cleaning auxiliaries such as bleach additives and "stain-stick" or pre-treat types. The terms "detergent composition" and "detergent formulation" are used in reference to mixtures which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to the variants according to the invention, the term encompasses detergents that may contain, e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, enzyme inhibitors or stabilizers, enzyme activators, transferase(s), hydrolytic enzymes, oxido reductases, bluing agents and fluorescent dyes, antioxidants, and solubilizers.

The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

The term "stability" includes storage stability and stability during use, e.g. during a wash process (in wash stability) and reflects the stability of the protease variant according to the invention as a function of time e.g. how much activity is retained when the protease is kept in solution, in particular in a detergent solution. The stability is influenced by many factors e.g. pH, temperature, detergent composition e.g. amount of builder, surfactants etc. The protease stability may be measured using the 'stability assay' as described in the Materials and Methods section herein. The term "improved stability" or "increased stability" is defined herein as a variant protease displaying an increased stability in solutions, relative to the stability of the parent protease. The terms "improved stability" and "increased stability" includes "improved chemical stability", "detergent stability" or "improved detergent stability.

### Detergent composition

The present invention relates to novel detergent compositions comprising bacterial mannanase enzymes. The detergent compositions comprising bacterial mannanases are useful in laundry and cleaning applications wherein degradation or modification of mannan is desired. The invention also relates to the use of said detergent compositions in laundry and cleaning applications as well as a method for degrading mannan.

As used herein, the term "mannan" refers to polysaccharides consisting of a mannose backbone linked together by β-1,4-linkages with side-chains of galactose attached to the backbone by α-1,6-linkages. Mannans comprise plant based material such as guar gum and locust bean gum. Glucomannans are polysaccharides having a backbone of more or less regularly alternating β-1,4 linked mannose and glucose, galactomannans and galactoglucomannans are mannans and glucomannans with alpha-1,6 linked galactose sidebranches.

As used herein, the term "mannanase" or "galactomannanase" denotes a mannanase enzyme defined according to the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78.

"Mannanase activity" as used herein refers to the mannan degrading activity of a polypeptide. Degrading or modifying as used herein means that mannose units are hydrolyzed from the mannan polysaccharide by the mannanase. The mannan degrading activity of the polypeptides according to present invention can be tested according to standard test procedures known in the art. Example 7 provides an example of a standard method for determining mannanase activity.

According to the first aspect the detergent composition of the present invention comprises at least one enzyme having an amino acid sequence having at least 93% sequence identity to the amino acid sequence of SEQ ID NO: 12 (Man6), wherein the at least one enzyme has mannan degrading activity.

In one embodiment of the invention the at least one enzyme has an amino acid sequence having at least 94%, at least 95%, at least 96%, at least 97%, at least 98%, or at least 99% sequence identity to the amino acid sequence of SEQ ID NO: 12.

The mannanases comprised in the detergent composition of the invention are suitable for degrading and modifying mannan containing material in various chemical environments, preferably in detergent compositions.

In one embodiment of the present invention the detergent composition further comprises one or more additional enzymes selected from the group consisting of protease, lipase, cutinase, amylase, carbohydrase, cellulase, pectinase, pectatlyase, mannanase, arabinase, galactanase, xylanase, oxidase, xanthanase, laccase, and/or peroxidase, preferably selected from the group consisting of proteases, amylases, cellulases and lipases.

In general the properties of the selected enzyme(s) should be compatible with the selected detergent, (i.e., pH-optimum, compatibility with other enzymatic and non-enzymatic ingredients, etc.), and the enzyme(s) should be present in effective amounts.

### Cellulases

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259. Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299._Example of cellulases exhibiting endo-beta-1,4-glucanase activity (EC 3.2.1.4) are those having described in WO02/099091._Other examples of cellulases include the family 45 cellulases described in WO96/29397, and especially variants thereof having substitution, insertion and/or deletion at one or more of the positions corresponding to the following positions in SEQ ID NO: 8 of WO 02/099091: 2, 4, 7, 8, 10, 13, 15, 19, 20, 21, 25, 26, 29, 32, 33, 34, 35, 37, 40, 42, 42a, 43, 44, 48, 53, 54, 55, 58, 59, 63, 64, 65, 66, 67, 70, 72, 76, 79, 80, 82, 84, 86, 88, 90, 91, 93, 95, 95d, 95h, 95j, 97, 100, 101, 102, 103, 113, 114, 117, 119, 121, 133, 136, 137, 138, 139, 140a, 141, 143a, 145, 146, 147, 150e, 150j, 151, 152, 153, 154, 155, 156, 157, 158, 159, 160c, 160e, 160k, 161, 162, 164, 165, 168, 170, 171, 172, 173, 175, 176, 178, 181, 183, 184, 185, 186, 188, 191, 192, 195, 196, 200, and/or 20, preferably selected among P19A, G20K, Q44K, N48E, Q119H or Q146 R._Commercially available cellulases include Celluclean^{™} Celluzyme^{™}, and Carezyme^{™} (Novozymes A/S), Clazinase^{™}, and Puradax HA^{™} (Genencor International Inc.), and KAC-500(B)^{™} (Kao Corporation).

### Proteases

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family. Examples of subtilases are those derived from *Bacillus* such as *Bacillus lentus, B. alkalophilus, B. subtilis, B. amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin 147* and *subtilisin 168* described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium* protease described in WO89/06270, WO94/25583 and WO05/040372, and the chymotrypsin proteases derived from *Cellumonas* described in WO05/052161 and WO05/052146. A further preferred protease is the alkaline protease from *Bacillus lentus* DSM 5483, as described for example in WO95/23221, and variants thereof, which are described in WO92/21760, WO95/23221, EP1921147 and EP1921148. Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering). Suitable commercially available protease enzymes include those sold under the trade names Alcalase^{®}, Duralase^{Tm}, Durazym^{Tm}, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®} and Esperase^{®} (Novozymes A/S), those sold under the tradename Maxatase^{®}, Maxacal^{®}, Maxapem^{®}, Purafect^{®}, Purafect Prime^{®}, Preferenz^{Tm}, Purafect MA^{®}, Purafect Ox^{®}, Purafect OxP^{®}, Puramax^{®}, Properase^{®}, Effectenz^{Tm}, FN2^{®}, FN3^{®} , FN4^{®}, Excellase^{®}, , Opticlean^{®} and Optimase^{®} (Danisco/DuPont), Axapem^{™} (Gist-Brocases N.V.), BLAP (sequence shown in Figure 29 of US5352604) and variants hereof (Henkel AG) and KAP (*Bacillus alkalophilus* subtilisin) from Kao.

### Lipases and Cutinases

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*)*,* e.g. *P*. *alcaligenes* or *P*. *pseudoalcaligenes* (EP218272), *P*. *cepacia* (EP331376), *P*. *sp.* strain SD705 (WO95/06720 & WO96/27002), *P*. *wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S*. *pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase^{™}, Lipex^{™}; Lipolex^{™} and Lipoclean^{™} (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

### Amylases

Suitable amylases which can be used together with subtilase variants of the invention may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 3 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity thereto. Preferred variants are those having a deletion in positions 181 and 182 and a substitution in position 193.

Other amylases, which are suitable are hybrid alpha-amylase comprising residues 1-33 of the alpha-amylase derived from *B*. *amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from *B*. *amyloliquefaciens* shown in SEQ ID NO: 6 of WO 2006/066594 and residues 36-483 of SEQ ID NO: 4 of WO2006/066594 are those having the substitutions:
M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184. Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T1311, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T1311+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl^{™}, Termamyl^{™}, Fungamyl^{™}, Stainzyme^{™}, Stainzyme Plus^{™}, Natalase^{™}, Liquozyme X and BAN^{™} (from Novozymes A/S), and Rapidase^{™} , Purastar^{™}/Effectenz^{™}, Powerase and Preferenz S100 (from Genencor International Inc./DuPont).

### Peroxidases/Oxidases

Suitable peroxidases/oxidases include those of plant, bacterial or fungal origin. Chemically modified or protein engineered mutants are included. Examples of useful peroxidases include peroxidases from *Coprinus, e.g.,* from *C. cinereus,* and variants thereof as those described in WO 93/24618, WO 95/10602, and WO 98/15257.

Commercially available peroxidases include Guardzyme^{™} (Novozymes A/S).

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.,* as disclosed in US 4,106,991 and 4,661,452 and may optionally be coated by methods known in the art. Examples of waxy coating materials are polyethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

A composition for use in solid laundry detergent, for example, may include 0.000001% - 5%, such as 0.000005%-2%, such as 0.00001%-1%, such as 0.00001%-0,1% of enzyme protein by weight of the composition.

A composition for use in laundry liquid, for example, may include 0.000001% - 3%, such as 0.000005 - 1%, such as 0.00001%-0,01% of enzyme protein by weight of the composition.

A composition for use in automatic dishwash, for example, may include 0.000001% - 5%, such as 0.000005%-2%, such as 0.00001%-1%, such as 0.00001%-0,1% of enzyme protein by weight of the composition.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in, for example, WO92/19709 and WO92/19708.

In one embodiment, the invention is directed to detergent compositions comprising an enzyme of the present invention in combination with one or more additional cleaning composition components. The choice of additional components is within the skill of the artisan and includes conventional ingredients, including the exemplary non-limiting components set forth below.

The choice of components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

### 1. Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl *N*-alkyl derivatives of glucosamine (glucamides, GA, or fatty acid glucamide, FAGA), as well as products available under the trade names SPAN and TWEEN, and combinations thereof.

When included therein the detergent will usually contain from about 0% to about 10% by weight of a semipolar surfactant. Non-limiting examples of semipolar surfactants include amine oxides (AO) such as alkyldimethylamineoxide, *N*-(coco alkyl)-*N*,*N*-dimethylamine oxide and *N*-(tallow-alkyl)-*N*,*N*-bis(2-hydroxyethyl)amine oxide, fatty acid alkanolamides and ethoxylated fatty acid alkanolamides, and combinations thereof.

When included therein the detergent will usually contain from about 0% to about 10% by weight of a zwitterionic surfactant. Non-limiting examples of zwitterionic surfactants include betaine, alkyldimethylbetaine, sulfobetaine, and combinations thereof.

### 2. Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### 3. Builders and Co-Builders

The detergent composition may contain about 0-65% by weight, such as about 5% to about 45% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg. Any builder and/or co-builder known in the art for use in laundry detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (*e.g*., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-20% by weight, such as about 5% to about 10%, of a detergent co-builder, or a mixture thereof. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N*'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra-(methylenephosphonic acid) (EDTMPA), diethylenetriaminepentakis(methylenephosphonic acid) (DTPMPA or DTMPA), *N-*(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N*, *N*-diacetic acid (α-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA) , taurine-*N*, *N*-diacetic acid (TUDA) and sulfomethyl-*N*, *N-*diacetic acid (SMDA), *N*-(2-hydroxyethyl)-ethylidenediamine-*N*, *N*', *N*'-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

### 4. Bleaching Systems

The detergent may contain 0-50% by weight, such as about 0.1% to about 25%, of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO98/17767. A particular family of bleach activators of interest was disclosed in EP624154 and particulary preferred in that family is acetyl triethyl citrate (ATC). ATC or a short chain triglyceride like triacetin has the advantage that it is environmental friendly as it eventually degrades into citric acid and alcohol. Furthermore acetyl triethyl citrate and triacetin has a good hydrolytical stability in the product upon storage and it is an efficient bleach activator. Finally ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae: (iii) and mixtures thereof; wherein each R¹ is independently a branched alkyl group containing from 9 to 24 carbons or linear alkyl group containing from 11 to 24 carbons, preferably each R¹ is independently a branched alkyl group containing from 9 to 18 carbons or linear alkyl group containing from 11 to 18 carbons, more preferably each R¹ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, e.g. in WO2007/087258, WO2007/087244, WO2007/087259 and WO2007/087242. Suitable photobleaches may for example be sulfonated zinc phthalocyanine

### 5. Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or polyethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of polyethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

### 6. Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

### 7. Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants: The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer Inhibiting Agents: The detergent compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent: The detergent compositions of the present invention will preferably also contain additional components that may tint articles being cleaned, such as fluorescent whitening agent or optical brighteners. Where present the brightener is preferably at a level of about 0,01% to about 0,5%.. Any fluorescent whitening agent suitable for use in a laundry detergent composition may be used in the composition of the present invention. The most commonly used fluorescent whitening agents are those belonging to the classes of diaminostilbene-sulphonic acid derivatives, diarylpyrazoline derivatives and bisphenyl-distyryl derivatives. Examples of the diaminostilbene-sulphonic acid derivative type of fluorescent whitening agents include the sodium salts of: 4,4'-bis-(2-diethanolamino-4-anilino-s-triazin-6-ylamino) stilbene-2,2'-disulphonate; 4,4'-bis-(2,4-dianilino-s-triazin-6-ylamino) stilbene-2.2'-disulphonate; 4,4'-bis-(2-anilino-4(N-methyl-N-2-hydroxy-ethylamino)-s-triazin-6-ylamino) stilbene-2,2'-disulphonate, 4,4'-bis-(4-phenyl-2,1,3-triazol-2-yl)stilbene-2,2'-disulphonate; 4,4'-bis-(2-anilino-4(1-methyl-2-hydroxy-ethylamino)-s-triazin-6-ylamino) stilbene-2,2'-disulphonate and 2-(stilbyl-4"-naptho-1.,2':4,5)-1,2,3-trizole-2"-sulphonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4 anilino-s-triazin-6-ylamino) stilbene disulphonate. Tinopal CBS is the disodium salt of 2,2'-bis-(phenyl-styryl) disulphonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins. Suitable fluorescent brightener levels include lower levels of from about 0.01, from 0.05, from about 0.1 or even from about 0.2 wt % to upper levels of 0.5 or even 0.75 wt%.

Soil release polymers: The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore random graft co-polymers are suitable soil release polymers Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents: The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

In a further embodiment of the present invention the detergent composition is in form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition. There are a number of detergent formulation forms such as layers (same or different phases), pouches, as well as forms for machine dosing unit.

Pouches can be configured as single or multicompartments. It can be of any form, shape and material which is suitable for hold the composition, e.g. without allowing the release of the composition from the pouch prior to water contact. The pouch is made from water soluble film which encloses an inner volume. Said inner volume can be devided into compartments of the pouch. Preferred films are polymeric materials preferably polymers which are formed into a film or sheet. Preferred polymers, copolymers or derivates therof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxyprpyl methyl cellulose (HPMC). Preferably the level of polymer in the film for example PVA is at least about 60%. Preferred average molecular weight will typically be about 20,000 to about 150,000. Films can also be of blend compositions comprising hydrolytically degradable and water soluble polymer blends such as polyactide and polyvinyl alcohol (known under the Trade reference M8630 as sold by Chris Craft In. Prod. Of Gary, Ind., US) plus plasticisers like glycerol, ethylene glycerol, Propylene glycol, sorbitol and mixtures thereof. The pouches can comprise a solid laundry detergent composition or part components and/or a liquid cleaning composition or part components separated by the water soluble film. The compartment for liquid components can be different in composition than compartments containing solids. Ref: (US2009/0011970 A1).

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

The detergent compositions of present invention may be provided in the form of laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change overtime, i.e. if a solid object (e.g. laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example Na⁺, K⁺ or NH₄⁺ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g a two stage vacuum plodder, extruders, cutters, logo-stampers, cooling tunnels and wrappers. The invention is not limited to preparing the laundry soap bars by any single method. The premix of the invention may be added to the soap at different stages of the process. For example, the premix containing a soap, an enzyme, optionally one or more additional enzymes, a protease inhibitor, and a salt of a monovalent cation and an organic anion may be prepared and and the mixture is then plodded. The enzyme and optional additional enzymes may be added at the same time as the protease inhibitor for example in liquid form. Besides the mixing step and the plodding step, the process may further comprise the steps of milling, extruding, cutting, stamping, cooling and/or wrapping.

The present invention furthermore relates to different uses of the detergent composition as herein disclosed, such as for degrading mannan and for use in a laundry process.

The present invention furthermore relates to a method for removing a stain from a surface, comprising contacting the surface with a detergent composition as herein disclosed.

The present invention also relates to a method for degrading mannan comprising applying a detergent composition as herein disclosed to mannan, preferably wherein the mannan is on the surface of a textile. By degrading mannan attached to the textile or fabric, dirt or soil bound to mannan is released and not capable of binding again to the mannan or mannan stains.

In an embodiment of the present invention the mannanase comprised in the detergent composition of present invention has a relative activity of at least 30% in the temperature range from 45° to 65° C. Providing mannanases that retain activity in temperatures above ambient temperature and in acidic pH is advantageous for applications wherein mannan degradation is required in such conditions.

In an embodiment the mannanase comprised in the detergent compositions of present invention hydrolyses endo-beta-1,4-mannosidic linkages randomly.

In an embodiment the mannanase comprised in the detergent compositions of present invention is obtainable or derivable from a bacterial source.

In an embodiment the mannanase comprised in the detergent compositions of present invention is fused with at least one further polypeptide, thus forming a fusion polypeptide. The fusion polypeptide or the further polypeptide may have other catalytic or binding activities in addition to those of mannanase. In an embodiment the further polypeptide comprises or consists of carbohydrate binding module, which is optionally a fragment of another protein or enzyme derived from the same or different organism as the mannanase. The mannanase can be connected to the further polypeptide with a linker.

### EXAMPLES

The following examples are provided to illustrate various aspects of the present invention. They are not intended to limit the invention, which is defined by the accompanying claims.

### Example 1: Screening

For identification of new beta-1,4-mannanases public databases (NCBI, EBI) and selected proprietary and public genomes were screened. All proprietary and public genomes used in this work are shown in Tab. 1. All hits were grouped and finally 15 genes of bacterial origin were selected for cloning in Bacillus based on the phylogenetic distance between each other (Table 2)

**Table 1: List of proprietary and public genomes used for screening of beta-1,4-mannanases**

| Species | Strain | Source |
|---|---|---|
| *Bacillus pumilus* | MS8 | ABE |
| *Amphibacillus xylanus* | NBRC 15112 | NCBI |
| *Bacillus hemicellulosilyticus* | JCM 9152 | NCBI |
| *Bacillus clausii* | KSM-K16 | NCBI |
| *Bacillus amyloliquefaciens* | RH1330 | ABE |
| *Virigibacillus soli* | PL205 | NCBI |

**Table 2: List of genes selected for cloning in Bacillus**

| **Sequence ID** | **Species** | **GH family** | **Length** |
|---|---|---|---|
| orf2511 | *Bacillus amyloliquefaciens* | 26 | 360 aa |
| AXY_08250 | *Amphibacillus xylanus* | 5 | 497 aa |
| Man7 | *Bacillus hemicellulosilyticus* | 5 | 490 aa |
| T1Z249.2 | *Bacillus nealsonii* | 5 | 369 aa |
| Man6 | *Bacillus clausii* | 5 | 324 aa |
| Q9EYQ3 | *Clostridium cellulolyticum* | 5 | 424 aa |
| YdhT | *Bacillus cellulosilyticus* | 26 | 1183 aa |
| V5X1N9 | *Paenibacillus polymyxa* | 5 | 588 aa |
| Q9ZI87 | *Geobacillus stearothermophilus* | 5 | 694 aa |
| Q49HI4 | *Bacillus circulans* | 5 | 327 aa |
| orf0659 | *Bacillus pumilus* | 5 | 376 aa |
| JCM9152_1090 | *Bacillus hemicellulosilyticus* | 26 | 489 aa |
| D3HC62 | *Streptococcus gallolyticus* | 5 | 487 aa |
| A0LSH9 | *Acidothermus cellulolyticus* | 5 | 763 aa |
| Man14 | *Virgibacillus soli* | 5 | 482 aa |

### Example 2: Cloning of bacterial mannanases in Bacillus

Unless otherwise stated, the molecular biological methods including DNA manipulations and transformations were performed as described in Sambrook and Russell (2001) and Harwood and Cutting (1990). The genes *man6,* man7and *man14* were amplified by PCR using Pfx Accu Prime Polymerase (Invitrogen). PCRs were performed according to manufacturer's instructions. Following PCR conditions were used for construction of the expression plasmids: 120 sec initial denaturation at 94 °C, followed by 35 cycles of 15 sec at 94 °C, 30 sec annealing at one of the following 50/55 °C, 110/290 sec extension at 68 °C and the final extension at 68 °C for 10 min. For amplification of *man7* genomic DNA of *Bacillus hemicellulosilyticus* JCM 9152 was used. *man6* and *man14* were ordered as synthetic genes without codon optimization (Eurofins MWG, Germany). Sequences of primers used for cloning are shown in Table 3. Overhangs for hybridization are underlined.

**Table 3: List of primers used for amplification of man6 (invention), man7 (referential) and man14 (referential)**

| **Template** | **Primer** | **bp** | **Sequence** | **Seq ID No** |
|---|---|---|---|---|
| syn. gene *man6* | Man6_1 | 39 | CAACCGCCTCTGCAGCTTATGCACAAA ACGGATTTCACG | 1 |
| syn. gene *man6* | Man6_2 | 39 | CGGTATATCTCTGTCTTAATCACTCTTA AGCCCATTTTC | 2 |
| gDNA *B. hemicellulosilyticus* | Man7_1 | 37 | CAACCGCCTCTGCAGCTTCTGATGGTC ATAGCCAAAC | 3 |
| gDNA *B. hemicellulosilyticus* | Man7_2 | 36 | CGGTATATCTCTGTCTTATTGGATTGTT ACATGATC | 4 |
| syn. Gene *man14* | Man14_1 | 40 | CAACCGCCTCTGCAGCTGCAAGCGGG TTTTATGTAAACGG | 5 |
| syn. Gene *man14* | Man14_2 | 39 | CGGTATATCTCTGTCTTATTTAATGGTA ACGTTATCAAC | 6 |
| pUB110 derivate | Vec_1 | 17 | AGCTGCAGAGGCGGTTG | 7 |
| pUB110 derivate | Vec_2 | 21 | GACAGAGATATACCGACAGTG | 8 |

Genes were cloned in a standard vector pEV1 pEV1 (Fig. 1), a pUB110 derivate including promoter PaprE from *Bacillus licheniformis* and xylanase signal peptide from *Bacillus amyloliquefaciens,* by using NEBuilder^{®}Hifi DNA Assembly Master Mix (NEB, Frankfurt). A vector:insert ration of 1:3 was applied for cloning. The total amount of fragments was at 0.2 pmol in a total volume of 20 µl. Samples were incubated for 40 min at 50°C. For construction purposes, expression plasmids were transformed by induced competence in *Bacillus subtilis* SCK6 as described in Zhang & Zhang 2011. The transformed cells were plated onto LB (Luria-Bertani) plates supplemented with 10 mg/l Kanamycin. Plates were incubated for 20h at 37°C. Arising colonies were picked and plasmid was isolated using QiaPrep MiniPrep Kit (Qiagen, Hilden). Isolation procedure was carried out according to the manufacturers recommendations for Gram positives plasmid preparations. Inserts were sequenced via Sanger sequencing (GATC, Germany) and revealed the DNA sequences corresponding to the mature parts of the mannanases Man6, Man7 and Man14. Sequence comparisons were done using ClustalW sequence alignment (Thompson et al 1994). Finally, expression plasmids were transformed in an appropriate *Bacillus* production strain via electroporation. *Bacillus* production strain was grown in electroporation medium containing 20g/l Trypton, 10g/l yeast extract, 10g NaCl and 2M saccharose and 10 ml were harvested at an OD(600nm) of 0.4. Cells were washed with electroporation buffer containing 0,272M saccharose, 1mM MgCl₂ and 7mM KH₂PO4 and finally resuspended in 250 µl electroporation buffer. Electroporation was performed using following conditions: 1,2 kV, 150 Ω, 50 µF. 1ml electroporation medium was added afterwards and cells were incubated for 3h at 37°C. Cells were plated on LB plates supplemented with 20 mg/l Kanamycin and incubated for 18h at 37°C. Clones were verified as described above and used for generation of material for analytic tests. Therefore, strains were inoculated in a standard expression under protein inducing conditions and incubated for 30h at 37°C. Supernatants were harvested and used for analytical and application tests. Genes and enzyme characteristics are shown in Table 4 and 5.

**Table 4. The summary on the GH5 family mannanase encoding genes from Bacillus clausii KSM-K16, Bacillus hemicellulosilyticus JCM 9152 and Virgibacillus soli PL205.**

| **Gene** | **Length including SP (bp)** | **SEQ ID NO** |
|---|---|---|
| *man6* | 975 | 9 |
| *man7* | 1473 | 13 |
| *man14* | 1449 | 17 |

**Table 5. The summary of the amino acid sequences deduced from the GH5 mannanase encoding gene sequences from Bacillus clausii KSM-K16, Bacillus hemicellulosilyticus JCM 9152 and Virgibacillus soli PL205.**

| Man protein | No of AAs | Length of SS | CBM | Predicted MW (Da), ss not included | Predicted pl, ss not included | SEQ ID NO |
|---|---|---|---|---|---|---|
| Man6 | 324 | 35 | | 31.84 | 4.56 | 11 |
| Man7 | 490 | 21 | Yes | 51.36 | 4.81 | 15 |
| Man14 | 482 | 16 | Yes | 50.68 | 4.35 | 19 |

### Example 3: PCR-cloning of bacterial mannanases man6 (invention) and man7 (referential) in Trichoderma reesei

Standard molecular biology methods were used in the isolation and enzyme treatments of DNA (e.g. isolation of plasmid DNA, digestion of DNA to produce DNA fragments), in *E. coli* transformations, sequencing etc. The basic methods used were either as described by the enzyme, reagent or kit manufacturer or as described in the standard molecular biology handbook, e.g. Sambrook and Russell (2001). Isolation of genomic DNA was performed as described in detail by Raeder and Broda (1985).

*Man6* and *man7* from *Bacillus clausii* and *Bacillus hemicellulosilyticus,* respectively, were also cloned for expression in *Trichoderma reesei* The genes were PCR-cloned using synthetic genes with codon optimization for *Trichoderma reesei.* DNA sequences encoding the signal peptides of *man6* and *man7* were removed using PCR and new cloning sites created. The sequences of the primers are shown in Table 6 (SEQ ID NOs: 21-24).

**Table 6. The oligonucleotides used as PCR primers to amplify Bacillus hemicellulosilyticus and Bacillus clausii mannanase genes.**

| Template, (synthetic) DNA from | Oligonucleotides | Length (bp) | Sequence^{(a} | SEQ ID NO: |
|---|---|---|---|---|
| *Bacillus hemicellulosilyticus* | BMAN1 | 60 | 5'- AGTCAATCGCGACAAGCGCC AGACCCACTCGGGCTTCTAC ATCGAGGGCTCGACGCTCTA- 3' (s) | 21 |
| *Bacillus hemicellulosilyticus* | BMAN2 | 46 | 5'- CGCGCCGGATCCTTACTGGA TCGTGACGTGGTCCAGGTAG ATGGCG-3' (as) | 22 |
| *Bacillus clausii* | BMAN3 | 60 | 5'- AGTCAATCGCGACAAGCGCC AGAACGGCTTCCACGTCTCC GGCACGGAGCTCCTGGACAA -3' (s) | 23 |
| *Bacillus clausii* | BMAN4 | 50 | 5'- CGCGCCGGATCCTTAGTCGC TCTTCAGGCCGTTCTCGCCG TAGACGATGCG-3' (as) | 24 |

| | | | | |
|---|---|---|---|---|
| ^{(a} "s" in the parenthesis = sense strand, "as" = antisense strand. | | | | |

The genes were amplified by PCR with primers described in Table 6 and using synthetic DNAs as templates in the reactions. The PCR mixtures of *Bacillus clausii man6* and *Bacillus hemicellulosilyticus man7* contained each 1x HF buffer for Phusion HF Polymerase (NEB/BioNordika, Finland), 0.2 mMdNTP mix (Thermo Fisher Scientific, Finland), 1 µM each primer, 3% DMSO (Thermo Fisher Scientific), 1 unit of Phusion High-Fidelity Polymerase (NEB/BioNordika, Finland) and 50 ng of the corresponding plasmid DNA. The conditions for the PCR reactions were the following: 30 sec initial denaturation at 98 °C, followed by 28 cycles of 10 sec at 98 °C, 30 sec annealing at one of the following 45/50/55/60 °C, 45 sec extension at 72 °C and the final extension at 72 °C for 7 min.

Primer combination described in Table 6 produced specific DNA products having the expected sizes. The PCR products were isolated from agarose gel with GenJet Gel Extraction Kit (Thermo Fisher Scientific) according to manufacturer's instructions, digested with *Nru*I and *Bam*HI restriction enzymes (Thermo Fisher Scientific) and cloned into an expression vector cleaved with *Nru*I and *Bam*HI. Ligation mixtures were transformed into *Escherichia coli* XL1-Blue (AH Diagnostics) and plated on LB (Luria-Bertani) plates containing 50-100 µg/ml ampicillin. Several *E. coli* colonies were collected from the plates and DNA was isolated with GenJet Plasmid Miniprep Kit (Thermo Fisher Scientific). Positive clones were screened using restriction digestions. The genes encoding the *Bacillus clausii man6* and *Bacillus hemicellulosilyticus man7* GH5 mannanases without their own signal peptide encoding sequences were sequenced and the plasmids were named pALK4274 and pALK4273, respectively (For details see Example 6).

### Example 4: Cloning of synthetic bacterial mannanase man14 (referential)

Standard molecular biology methods were used in the isolation and enzyme treatments of DNA (e.g. isolation of plasmid DNA, digestion of DNA to produce DNA fragments), in *E. coli* transformations, sequencing etc. The basic methods used were either as described by the enzyme, reagent or kit manufacturer or as described in the standard molecular biology handbook, e.g. Sambrook and Russell (2001). Isolation of genomic DNA was performed as described in detail by Raeder and Broda (1985).

Mannanase gene *man14* from *Virgibacillus soli* was also cloned for *Trichoderma* expression as well. The gene encoding GH5 family mannanase Man14 from *Virgibacillus soli* was ordered from GenScript as a synthetic construct with codon optimization for *Trichoderma reesei.*

Plasmid DNA obtained from GenScript including the *man14* gene was re-suspended in sterile water, digested with *Nru*I and *Bam*HI restriction enzymes (Thermo Fisher Scientific) according to manufacturer's instructions and cloned into an expression vector cleaved with *Nru*I and *Bam*HI. Ligation mixture was transformed into *Escherichia coli* XL1-Blue (AH Diagnostics) and plated on LB (Luria-Bertani) plates containing 50-100 µg/ml ampicillin. Several *E. coli* colonies were collected from the plates and DNA was isolated with GenJet Plasmid Miniprep Kit (Thermo Fisher Scientific). Positive clones were screened using restriction digestions and they were shown to contain inserts of expected sizes. Fusion sites of *Virgibacillus soli man14* to the expression plasmid were sequenced and the plasmid was named pALK4414 (For details see Example 6).

### Example 5: Production of recombinant bacterial GH5 mannanase proteins in Bacillus

Expression plasmids were constructed for production of recombinant GH5 mannanase (Man6 (invention), Man7 (referential) and Man14 (referential)) proteins from *Bacillus clausii, Bacillus hemicellulosilyticus* and *Virgibacillus soli.* The expression plasmids constructed are listed in Table 7. The recombinant GH5 genes (*man*6*, man*7 and *man*14), without their own signal sequences, were fused to the *Bacillus licheniformis PaprE promoter* and *B*. *amyloliquefaciens* xylanase signal peptide. The transcription termination was ensured by a strong terminator and a kanamycin resistance marker was used for selection of the transformants. The transformations were performed as described in Example 2.

**Table 7. The expression plasmids constructed to produce Man6, Man7 and Man14 recombinant proteins from Bacillus clausii, Bacillus hemicellulosilyticus and Virgibacillus soli in an appropriate Bacillus expression strain.**

| **Mannanase (GH5) protein** | **Expression plasmid** |
|---|---|
| Man6 | pEV1 Man6 |
| Man7 | pEV1 Man7 |
| Man14 | pEV1 Man14 |

The GH5 production of the transformants was analyzed from the culture supernatants of the shake flask cultivations. The transformants were inoculated from the LB plates to shake flasks containing 2% glucose, 6% corn steep powder, 1,3 % (NH4)2HPO4 , 0,05% MgSO4 x 7H2O and 0,5% CaCl2. pH was adjusted to pH 7.5. The GH5 protein production of the transformants was analyzed from culture supernatants after growing them for 30 hours at 37 °C, 180 rpm. Heterologous production of recombinant proteins was analyzed by SDS-PAGE with subsequent Coomassie staining. The best producing transformants were chosen to be cultivated in laboratory scale bioreactors. The transformants were cultivated in bioreactors at 37 °C under protein inducing conditions and additional feeding until a suitable yield was reached. The supernatants were recovered for application tests by centrifugation or filtration.

### Example 6: Production of recombinant bacterial GH5 mannanase proteins in Trichoderma reesei

Expression plasmids were constructed for production of recombinant GH5 mannanase (Man6 (invention), Man7 (referential) and Man14 (referential)) proteins from *Bacillus clausii, Bacillus hemicellulosilyticus* and *Virgibacillus soli* (See Examples 3 and 4) in *Trichoderma reesei.* The expression plasmids constructed are listed in Table 8. The recombinant GH5 genes (*man6, man7* and *man14*)*,* without their own signal sequences, were fused to the *T. reesei cel7A*/*cbh1* promoter with *T*. *reesei cel6*A/*cbh2* CBM carrier and linker followed by Kex2 protease recognition site. The transcription termination was ensured by the *T. reesei cel7A*/*cbh1* terminator and the *A*. *nidulans amd*S marker gene was used for selection of the transformants as described in Paloheimo *et al.* (2003). The linear expression cassettes (Fig. 2) were isolated from the vector backbones after *Not*I digestions and were transformed into *T*. *reesei* protoplasts. The host strains used does not produce any of the four major *T*. *reesei* cellulases (CBHI, CBHII, EGI, EGII). The transformations were performed as in Penttilä *et al.* (1987) with the modifications described in Karhunen *et al.* (1993), selecting acetamidase as a sole nitrogen source (*amd*S marker gene). The transformants were purified on selection plates through single conidia prior to sporulating them on PD.

**Table 8. The expression cassettes constructed to produce Man6, Man7 and Man14 recombinant proteins from Bacillus clausii, Bacillus hemicellulosilyticus and Virgibacillus soli in Trichoderma reesei. The overall structure of the expression cassettes was as described in Fig. 2.**

| **Mannanase (GH5) protein** | **Expression plasmid** | **Expression cassette ^{(a}** |
|---|---|---|
| Man6 | pALK4274 | 7.0 kb *Not*I |
| Man7 | pALK4273 | 7.5 kb *Not*I |
| Man14 | pALK4414 | 7.6 kb *Not*I |

| | | |
|---|---|---|
| ^{(a} The expression cassette for *T*. *reesei* transformation was isolated from vector backbone by using *Not*I digestion. | | |

The mannanase production of the transformants was analyzed from the culture supernatants of the shake flask cultivations. The transformants were inoculated from the PD slants to shake flasks containing 50 ml of complex lactose-based cellulase inducing medium (Joutsjoki *at al.* 1993) buffered with 5% KH₂PO₄. The GH5 protein production of the transformants was analyzed from culture supernatants after growing them for 7 days at 30 °C, 250 rpm. Heterologous production of recombinant proteins was analyzed by SDS-PAGE with subsequent Coomassie staining. The best producing transformants were chosen to be cultivated in laboratory scale bioreactors. The transformants were cultivated in bioreactors either on batch or by additional feeding type of process under protein inducing conditions at a typical mesophilic fungal cultivation temperature and slightly acidic conditions. The cultivation was continued until depletion of the medium sugars or until suitable yield was reached. The supernatants were recovered for application tests by centrifugation or by filtration.

### Example 7: Assay of galactomannanase activity by DNS -method

Mannanase activity (MNU) was measured as the release of reducing sugars from galactomannan (0.3 w/w-%) at 50°C and pH 7.0 in 5 min. The amount of released reducing carbohydrates was determined spectrophotometrically using dinitrosalicylic acid. Substrate (0,3 w/w-%) used in the assay was prepared as follows: 0.6 g of locust bean gum (Sigma G-0753) was in 50 mM sodium citrate buffer pH 7 (or citrate phosphate buffer pH 7) at about 80 °C using a heating magnetic stirrer and heated up to boiling point. The solution was cooled and let to dissolve overnight in a cold room (2 - 8 °C) with continuous stirring and insoluble residues were removed by centrifugation. After that solution was filled up to 200 ml by buffer. Substrate was stored as frozen and melted by heating in a boiling water bath to about 80 °C, cooled to room temperature and mixed carefully before use. DNS reagent used in the assay was prepared by dissolving 50 g of 3.5- dinitrosalisylic acid (Sigma D-550) in about 4 liter of water. With continuous magnetic stirring 80.0 g of NaOH was gradually added and let to dissolve. An amount of 1500 g of Rochelle Salt (K-Na-tartrate, Merck 8087) was added in small portions with continuous stirring. The solution that might have been cautiously warmed to a maximum temperature of 45 °C., was cooled to room temperature and filled up to 5000 ml. After that it was filtered through Whatman 1 filter paper and stored in a dark bottle at room temperature. The reaction was first started by adding 1.8 ml of substrate solution to each of the two test tubes and let to equilibrate at 50 °C for 5 minutes, after which 200 µl of suitably diluted enzyme solution was added to one of the tubes, mixed well with vortex mixer and incubated exactly for 5 min at 50 °C. Enzyme blanks didn't need to be equilibrated or incubated. The reaction was stopped by adding 3.0 ml of DNS reagent into both tubes and mixed. 200 µl of sample solution was added to the enzyme blank tubes. Both tubes were placed in a boiling water bath. After boiling for exactly 5 minutes, the tubes were placed in a cooling water bath and allow them to cool to room temperature. The absorbance of sample was measured against the enzyme blank at 540 nm and activity was read from the calibration curve and multiplied by the dilution factor. A suitable diluted sample yielded an absorbance difference of 0.15 - 0.4. Standard curve was prepared 20 mM from mannose stock solution by dissolving 360 mg of mannose (SigmaM-6020, stored in a desiccator) in assay buffer and diluted to solutions containing 3, 6, 10 and 14 µmol/ml of mannose. Standards were handled like the samples except for incubating at 50°C. The absorbances were measured against the reagent blank (containing buffer instead of standard dilution of mannose) at 540nm. Calibration curve was constructed for every series of assays. One mannanase unit (MNU) was defined as the amount of enzyme that produces reductive carbohydrates having a reductive power corresponding to one nmol of mannose from galactomannan in one second under the assay conditions (1 MNU = 1nkat).

### Example 8: Purification of Man6 mannanase

Cells and solids were removed from the fermentation culture medium by centrifugation for 10 min, 4000 g at 4°C. The supernatant of 10 ml was used for protein purification. The sample was filtered through 0.44 µm PVDF membrane (Millex-HV, Merck Millipore Ltd,Carrigtwohill, IRL). The filtrate was loaded onto a HiPrep 26/10 Desalting column (GE Healthcare, Uppsala, Sweden) equilibrated in 20 mM HEPES pH 7. The desalted sample was then loaded onto a 5 ml HiTrap Q HP column (GE Healthcare, Uppsala, Sweden) pre-equilibrated with 20 mM HEPES pH 7. After sample loading, the column was washed with the same buffer for 20 ml. Proteins were eluted with linear salt gradient 20 mM HEPES, 500 mM NaCl pH 7 in 15 CVs. Fractions of 5 ml were collected and analyzed on SDS-PAGE. The fractions containing target protein were combined and concentrated to 2 ml using Vivaspin 20, 10 kDa MWCO ultrafiltration devices (GE Healthcare). The concentrated sample was further fractionated using Superdex 75 26/60 gel-filtration column equilibrated with 20 mM MES, 200 mM NaCl pH 6,5. Fractions of 2 ml were collected and analyzed by SDS-PAGE. Fractions containing pure mannanase were combined. Other mannanases were purified using the same protocol but changing the buffer composition in desalting and ion exchange steps. Buffer compositions are shown in Table 9.

**Table 9. Buffers used in ion exchange chromatography**

| Mannanase | Buffers used in ion exchange chromatography |
|---|---|
| Man6 | 20 mM HEPES pH 7 |
| Man7 | 20 mM HEPES pH 7 |
| Man14 | 20 mM MES pH 6 |

| | |
|---|---|
| Purified samples were above 95% pure. | |

Enzyme content of the purified sample was determined using UV absorbance 280 nm measurements. Excitation coefficients for each mannanases were calculated on the bases of amino acid sequence of the enzyme by using ExPASy Server http://web.expasy.org/protparam/. (Gasteiger E et al 2005). The enzyme activity (MNU) of purified samples was measured as release of reducing sugars as described in Example 7. The specific activity (MNU/mg) of mannanases was calculated by dividing MNU activity of purified sample with the amount of purified enzyme. Obtained values were used for calculating enzyme dosages used in Examples 10 and 11.

### pH profiles of mannanases

The pH profiles of purified mannanases were determined using the beta-mannazyme tablet assay Azurine-crosslinked carob galactomannan (T-MNZ 11/14) from Megazyme with minor modifications to the suggested protocol. The linearity of the assay has been checked with each purified enzymes. The assay was performed in 40 mM Britton-Robinson buffer adjusted to pH values between 4 and 11. The enzyme solution was diluted into the assay buffer and 500µl of enzyme solution was equilibrated at 50C water bath for 5 min before adding one substrate tablet. After 10 minutes, the reaction was stopped by adding 10 ml 2% Tris pH 12. The reaction tubes were left at room temperature for 5 min, stirred and the liquid filtered through a Whatman No.1 paper filter. Release of blue dye from the substrate was quantified by measuring the absorbance at 595 nm. Enzyme activity at each pH was reported as relative activity where the activity at the pH optimum was set to 100%. The pH profiles were shown in Figure 3.

Relative activity (%) of mannanase is calculated by dividing mannanase activity of a sample by the mannanase activity of a reference sample. In the case of pH profile, the reference sample is a sample at the optimal pH. In the case of temperature profile the reference sample is a sample at the optimal temperature.

### Temperature profiles of mannanases

The temperature optimum of purified mannanases was determined using the beta-mannazyme tablet assay Azurine-crosslinked carob galactomannan (T-MNZ 11/14) from Megazyme with minor modifications to suggested protocol. The assay was performed at temperatures varying between 30-90°C for 10 minutes in 40 mM Britton-Robinson buffer pH7. Enzyme activity was reported as relative activity where the activity at temperature optimum was set to 100%. The temperature profiles were shown in Figure 4.

### Temperature and pH characteristics of mannanases

Man6 has a molecular mass between 30-35 kDa. The optimal temperature of the enzyme at pH 7 is from 50°C to 70°C. Said enzyme has pH optimum at the pH range of at least pH 6 to pH 9 at 50°C. The optimal temperature and pH optimum were determined using 10 min reaction time and Azurine-crosslinked carob galactomannan as a substrate.

Man7 has a molecular mass between 50-55 kDa. The optimal temperature of the enzyme at pH 7 is from 50°C to 70°C. Said enzyme has pH optimum at the pH range of at least pH 7 to pH 10 at 50°C. The optimal temperature and pH optimum were determined using 10 min reaction time and Azurine-crosslinked carob galactomannan as a substrate.

Man14 has a molecular mass between 30-40 kDa. The optimal temperature of the enzyme at pH 7 is from 50°C to 70°C. Said enzyme has pH optimum at the pH range of at least pH 7 to pH 8 at 50°C. The optimal temperature and pH optimum were determined using 10 min reaction time and Azurine-crosslinked carob galactomannan as a substrate.

### Example 9: Stain removal performance of Man6 (invention) and Man7 (referential) mannanases with commercial detergents without bleaching agents

Man6 and Man7 mannanases produced in *Bacillus* (as described in Example 5) and in *Trichoderma* (as described in Example 6), were tested for their ability to remove mannanase sensitive standard stains at 40 °C and water hardness of 16°dH with commercial detergents without bleaching agents and compared to commercial mannanase preparation Mannaway^{®} 4,0 L (Novozymes). The following artificially soiled test cloths from Center for testmaterial B.V. (the Netherlands) were used: Chocolate pudding mannanase sensitive on cotton (E-165), Locust bean gum, with pigment on cotton (C-S-73) and on polyester/cotton (PC-S-73) and Guar gum with carbon black on cotton (C-S-43). The fabric was cut in 6 cm x 6 cm swatches and 2 pieces of each were used in test.

Commercial heavyduty liquid detergent Acontaining all other enzymes except mannanase was used at concentration of 4.4 g per liter of wash liquor and Commercial Color detergent powder without enzymes was used at 3.8 g/l. Detergent containing wash liquors we prepared in synthetic tap water with hardness of 16 °dH. Protease Savinase^{®} 16 L (0.5 w/w % ) and amylase Stainzyme^{®} 12 L (0.4 w/w %) was added into hard water used with commercial Color detergent powder, the liquid detergent already contained amylase and protease. pH of the wash liquor of Color detergent powder was approximately 10 and with the liquid detergent approximately 8.3.

Mannanase dosages were in range 0 - 0.2/0.25% of detergent weight but for the evaluation the dosages were calculated as enzyme activity units (MNU) per ml wash liquor or as mg of active enzyme protein (AEP) per I of wash liquor. Activity was measured as described in Example 7. AEP content of each preparation was calculated by dividing the enzyme activity with specific activity, defined in Example 8. Control sample contained the detergent solution but no mannanase.

For synthetic tap water with hardness of 16 °dH the following stock solutions were prepared in deionized water (Milli-Q or equivalent):
Stock solution with 1000 °d Calcium-hardness: CaCl2 x 2 H2O (1.02382.1000, Merck KGaA, Germany) 26,22 g/I
Stock solution with 200 °d Magnesium-hardness: MgSO4 x 7 H2O (1.05886.1000, Merck KGaA, Germany) 8,79 g/l H2O
NaHCO3 stock solution: NaHCO3 (1.06329.1000 Merck KGaA, Germany) 29,6 g/l
13,3 ml CaCl2 solution, 13,3 ml MgSO4 solution and 10,0 ml of freshly made NaHCO3 solution were added in volumetric flask in the given order, made up to 1 liter with deionized water and mixed. The hardness of water was determined by complexometric titration and found correct.

Stain removal treatments were performed in Atlas LP-2 Launder-Ometer as follows. Launder-Ometer was first preheated to 40°C. Then detergent, 250 ml synthetic tap water with hardness of 16 °dH and diluted enzyme (<1,0 ml) were added into 1,2 liter containers. Stains were added and the Launder-Ometer was run at 40°C for 60 min with a rotation speed of 42 rpm. After that the swatches were carefully rinsed under running water and dried overnight at indoor air, on a grid protected against daylight. The stain removal effect was evaluated by measuring the colour as reflectance values with Konica Minolta CM-3610A spectrophotometer using L*a*b* color space coordinates (illuminant D65/10°, 420 nm cut). Fading of the stains, indicating mannanase performance (stain removal efficiency) was calculated as ΔL* (delta L*), which means lightness value L* of enzyme treated fabric minus lightness value L* of fabric treated with washing liquor without mannanase (control). Final results (total stain removal effect) were shown as sum of ΔL* of each stains. Color values of each stains were average of 2 swatches. The results obtained with commercial liquid detergent are shown in Figs. 6-7. The mannanases according to the invention have similar (Man6) or considerably better (Man7) stain removal performance with liquid detergent when dosed as activity units or as active enzyme protein compared to commercial mannanase preparation Mannaway^{®} 4,0 L. Similar performance was obtained with Man6 and Man7 regardless of the expression host, *Bacillus* or *Trichoderma* (Fig 6). The results obtained with commercial color detergent powder (Figs. 8-9) show that the mannanases according to the invention have better stain removal performance with color detergent powder when dosed as activity units or as active enzyme protein compared to commercial mannanase preparation Mannaway^{®} 4,0 L.

### Example 10: Stain removal performance Man6 (invention) and Man7 (referential) mannanases with bleach containing detergent

Man6 and Man7 mannanases produced in *Bacillus* (as described in Example 5) were tested for their ability to remove mannanase sensitive standard stains at 40 °C and water hardness of 16°dH with commercial bleach detergent powder and compared to commercial mannanase preparation Mannaway^{®} 4,0 L (Novozymes). Test system was similar to described in Example 9, except 3 different artificially soiled test cloths from Center for testmaterial B.V. (the Netherlands) were used: Chocolate pudding mannanase sensitive on cotton (E-165), Locust bean gum, with pigment on cotton (C-S-73) and Guar gum with carbon black on cotton (C-S-43). Commercial Color detergent powder was used at concentration of 4,2 g per liter of wash liquor and pH of the wash liquor was approx. 9,5. Protease Savinase^{®} 16 L (0.5 w/w %) and amylase Stainzyme^{®} 12 L (0.4 w/w %) were added into hard water used in test, since the detergent didn't contain any enzymes. The color of the swatches after treatment was measured and results calculated as sum of ΔL* of each 3 stains as described in Example 9. The results (Fig. 10) obtained with commercial bleach containing detergent indicate that the mannanase according to the invention (Man7) has considerably better stain removal performance compared to commercial mannanase Mannaway^{®} 4,0 L when dosed as active enzyme protein. With Man6 at least similar performance compared to a commercial bacterial mannanase is obtained.

### Example 11: Stain removal performance Man14 (referential) mannanase with commercial liquid detergent

Man14 mannanase produced in Bacillus (as described in Example 5) was tested for their ability to remove mannanase sensitive standard stains at 40 °C and water hardness of 16°dH with commercial heavy duty liquid detergent B and compared to commercial mannanase preparation Mannaway^{®} 4,0 L (Novozymes). Test system was similar to that described in Example 9, except two different artificially soiled test cloths from Center for testmaterial B.V. (the Netherlands) were used: Chocolate pudding mannanase sensitive on cotton (E-165) and Locust bean gum, with pigment on cotton (C-S-73). Commercial heavy duty liquid detergent B was used at concentration of 5 g per liter of wash liquor and pH of the wash liquor was approx. 8.3. Protease Savinase^{®} 16 L (0,5 w/w %) and amylase Stainzyme^{®} 12 L (0,4 w/w %) were added into hard water used in test, since the detergent didn't contain any enzymes. The color of the swatches after treatment was measured and results calculated as sum of ΔL* of each 2 stains as described in Example 9. The results (Figs. 11-12) obtained with commercial liquid containing detergent indicate Man14 had good performance in a liquid detergent, comparable to commercial product, when dosed either as activity units or as active enzyme protein.

### Example 12: Stability of Man6 (invention) and Man7 (referential) mannanases in commercial liquid detergents

The stability of Man6 and Man7 mannanase preparations produced in *Bacillus* were tested in OMO Color liquid obtained from local super market and compared to commercial mannanase preparation Mannaway^{®} 4,0 L. Mannanase preparations were added 0,5 % w/w-% in detergents and samples were incubated in plastic tubes with caps at 37°C for 5 weeks. The activity was measured at certain intervals by activity assay described in Example 7 except using 30 min incubation time. Results were calculated as residual activity (%), which was obtained by dividing the activity of a sample taken at certain time point by initial activity of the sample. The stability of Man7 produced both in *Bacillus* and *Trichoderma* and Man6 produced in *Trichoderma* were tested against Mannaway^{®} 4,0 L also in commercial liquid heavyduty detergent A containing protease but no mannanase. In this test 1 % -(w/w) of mannanases were used and samples incubated for 37°C for 12 weeks. The results in Omo Color (Fig. 13) show that Man6 had considerably better and Man7 similar stability compared to Mannaway^{®} 4,0 L Both Man7 and especially Man6 were more stable than Mannaway^{®} 4,0 L with another commercial liquid detergent A, as shown in Fig 14. Results obtained in another test at same conditions showed that Man6 had similar stability regardless of the expression host, *Bacillus* or *Trichoderma* (data not shown). The results of the stability experiments show that the mannanase according to the invention is stabile in detergents for several weeks even when stored at high temperature like 37°C. The stability of the mannanases according to the invention (Man6 and Man7) is improved compared to a commercial bacterial mannanase in liquid detergent.

### Example 13: Wash performance of liquid detergent compositions according to the invention

The wash performance of liquid detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") and Warwick Equest Ltd Unit 55, Consett Business Park, Consett, County Durham ("Equest").

A liquid washing agent with the following composition was used as base formulation (all values in weight percent):

| Chemical name | Active substance raw material [%] | Active substance detergent formulation [%] |
|---|---|---|
| Water demin. | 100 | Rest |
| Alkyl benzene sulfonic acid | 96 | 2-7 |
| Anionic surfactants | 70 | 6-10 |
| C12-C18 Fatty acid sodium salt | 30 | 1-4 |
| Nonionic surfactants | 100 | 4-7 |
| Phosphonates | 40 | 0,1-2 |
| Citric acid | 100 | 1-3 |
| NaOH | 50 | 1-4 |
| Boronic acid | 100 | 0,1-2 |
| Antifoaming agent | 100 | 0,01-1 |
| Glycerol | 100 | 1-3 |
| Enzymes | 100 | 0,1-2 |
| Preserving agent | 100 | 0,05-1 |
| Ethanol | 93 | 0,5-2 |
| Optical brightener | 90 | 0,01-1 |
| Perfume | 100 | 0,1-1 |
| Dye | 100 | 0,001-0,1 |

The pH of the detergent composition was between 8,2-8,6.

Based on this base formulation, liquid detergent compositions 1 and 2 were prepared by adding respective enzymes as indicated below:
Composition 1: Enzyme according to SEQ ID NO:12 (Man6) (invention)
Composition 2: Enzyme according to SEQ ID NO:16 (Man7) (referential)

The wash was performed as follows according to the AISE Method: 3,5 kg Clean ballast cloth, 4 SBL Cloths, Miele washing machine, 20°C and 40°C Short program.

All mannanases were added in the same amounts based on total protein content.

The dosing ratio of the liquid washing agent was 4.0 grams per liter of washing liquor. The washing procedure was performed for 60 minutes at a temperature of 20°C and 40°C, the water having a water hardness between 15.5 and 16.5° (German degrees of hardness).

The results obtained are the difference values between the remission units obtained with the detergents and the remission units obtained with the detergent containing the commercially available reference mannanase (Mannaway 4.0L, obtained from Novozymes). A positive value therefore indicates an improved wash performance of the detergent compositions comprising the mannanases of present invention compared to the same detergent composition comprising the reference mannanase. Within the washing test a large range of stains were tested.

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the enzyme combinations. A positive value therefore indicates an improved wash performance due to the enzyme combinations present in the detergent. Mannanases of the invention in detergent compositions show improved performance on a variety of mannan comprising stains.

| | 20°C | | 40°C | |
|---|---|---|---|---|
| Stain | Comp.1 | Comp.2 | Comp.1 | Comp.2 |
| Chocolate Ice Cream (Equest) | 1.3 | 4.2 | n.d. | n.d. |
| Carte Dór Chocolate Ice Cream (Equest) | n.d. | 3.3 | n.d. | 0.7 |
| Cocoa [CO] (Equest) | n.d. | 2.3 | n.d. | n.d. |
| Mayonnaise/Carbon black color (CFT CS05S [CO]) | 1.3 | 4.3 | 1.1 | 2.2 |
| Salad dressing, with natural black (CFT CS06 [CO]) | 1.2 | 3.5 | 1.2 | 2,6 |
| Lipstick, diluted, Red (CFT CS216 [CO]) | n.d. | 1.5 | n.d. | 0.7 |

### Example 14: Wash performance of powder detergent compositions according to the invention

The wash performance of powder detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") Warwick Equest Ltd Unit 55, Consett Business Park, Consett, County Durham ("Equest").

A solid washing agent with the following composition was used as base formulation (all values in weight percent):

| Chemical Name | Active substance raw material [%] | Active substance detergent formulation [%] |
|---|---|---|
| Water demin. | 100 | 1-4 |
| Alkyl benzene sulfonic acid | 97 | 9-13 |
| Nonionic surfactants | 100 | 4-7 |
| Percarbonates | 88 | 9-13 |
| TAED | 92 | 1-5 |
| Phosphonates | 60 | 0,1-3 |
| Polyacrylates | 45 | 1-4 |
| Soduim silicate | 40 | 5-10 |
| Sodium carbonate | 100 | 18-22 |
| Carboxymethylcellulose | 69 | 1-4 |
| Soil release polymer | 100 | 0,1-1 |
| Optical brightener | 70 | 0,1-1 |
| Antifoaming agent | t.q. | 0,01-1 |
| Sodium sulfate | 100 | 22-30 |
| Enzymes | 100 | 0,1-1 |
| Perfume | 100 | 0,1-1 |
| NaOH | 100 | 0,1-1 |
| Rest | - | 1-4 |

Based on this base formulation, solid detergent compositions 3 and 4 were prepared by adding respective enzymes as indicated below:
Composition 3: Enzyme according to SEQ ID NO:12 (Man6) (invention)
Composition 4: Enzyme according to SEQ ID NO:16 (Man7) (referential)

The wash was performed as follows according to the AISE Method: 3,5 kg Clean ballast cloth, 4 SBL Cloths, Miele washing machine, 20°C and 40°C Short program. All mannanases were added in the same amounts based on total protein content.

The dosing ratio of the powder washing agent was 3.8 grams per liter of washing liquor. The washing procedure was performed for 60 minutes at a temperature of 20°C and 40°C, the water having a water hardness between 15.5 and 16.5° (German degrees of hardness).

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the reference mannanase (Mannaway 4.0L, obtained from Novozymes). A positive value therefore indicates an improved wash performance of the variants in the detergent. Mannanases of the invention show improved performance on several stains. Therefore, it is evident that mannanases according to the invention show improved wash performance compared to Mannaway.

| | 20°C | | 40°C | |
|---|---|---|---|---|
| Stain | Comp.3 | Comp.4 | Comp.3 | Comp.4 |
| Carte Dór Chocolate Ice Cream (Equest) | 1,4 | 2,8 | 2,1 | 0,5 |
| Vienetta (Equest) | 0,5 | 0,8 | 0,5 | n.d. |
| Chocolate Icecream L [CO] (Equest) | 0,9 | 0,9 | 1,1 | n.d. |
| Porridge (EMPA 163 [CO]) | n.d. | n.d. | 1,3 | 5,1 |
| Cocoa (CFT CS02 [CO]) | 1,8 | 3,1 | n.d. | n.d. |
| Mayonnaise/Carbon black color (CFT CS05S [CO]) | n.d. | 1,0 | n.d. | 2,7 |
| Salad dressing, with natural black (CFT CS06 [CO]) | 2,0 | 4,8 | 1,3 | 5,1 |
| Sebum BEY with carbon black (CFT CS32 [CO]) | 0,7 | 1,4 | 0,5 | 0,7 |
| Chocolate drink, pure (CFT CS44 [CO]) | n.d. | 1,4 | n.d. | 0,8 |

Without limiting the scope and interpretation of the patent claims, certain technical effects of one or more of the aspects or embodiments disclosed herein are listed in the following: A technical effect is degradation or modification of mannan. Another technical effect is provision of mannanase which has good storage stability. The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented above, but that it can be implemented in other embodiments using equivalent means without deviating from the characteristics of the invention.

## Claims

1. Detergent composition comprising at least one enzyme having an amino acid sequence having at least 95% sequence identity to the amino acid sequence of SEQ ID NO: 12, Wherein the at least one enzyme has mannan degrading activity.

2. Detergent composition of claim 1, wherein the at least one enzyme has an amino acid sequence having at least 96%, at least 97%, at least 98%, or at least 99% sequence identity to the amino acid sequence of SEQ ID NO: 12.

3. The detergent composition of any one of claims 1 to 2, the composition further comprising one or more additional enzymes selected from the group consisting of protease, lipase, cutinase, amylase, carbohydrase, cellulase, pectinase, pectatlyase, mannanase, arabinase, galactanase, xylanase, oxidase, xanthanase, laccase, and/or peroxidase.

4. The detergent composition according to any one of claims 1 to 3, wherein the composition is in form of a bar, a homogenous tablet, a tablet having two or more layers, a pouch having one or more compartments, a regular or compact powder, a granule, a paste, a gel, or a regular, compact or concentrated liquid.

5. The detergent composition of any one of claims 1 to 4, wherein the detergent composition is a laundry detergent composition, preferably a liquid or solid laundry detergent composition.

6. Use of a detergent composition of any of claims 1 to 5 for degrading mannan.

7. Use of a detergent composition according to any one of claims 1 to 5 in a laundry process.

8. A method for removing a stain from a surface, comprising contacting the surface with a detergent composition according to any one of claims 1 to 5.

9. A method for degrading mannan comprising applying a detergent composition according to any of claims 1 to 5 to mannan, preferably wherein the mannan is on the surface of a textile.

## Patentansprüche

1. Detergenszusammensetzung, umfassend mindestens ein Enzym mit einer Aminosäuresequenz, die mindestens 95 % Sequenzidentität mit der Aminosäuresequenz von S E Q ID NO: 12 aufweist, wobei das mindestens eine Enzym eine Mannan-abbauende Aktivität aufweist.

2. Detergenszusammensetzung nach Anspruch 1, wobei das mindestens eine Enzym eine Aminosäuresequenz mit mindestens 96 %, mindestens 97 %, mindestens 98 % oder mindestens 99 % Sequenzidentität mit der Aminosäuresequenz von SEQ ID NO: 12 aufweist.

3. Detergenszusammensetzung nach einem der Ansprüche 1 bis 2, wobei die Zusammensetzung ferner ein oder mehrere zusätzliche Enzyme umfasst, das/die aus der Gruppe ausgewählt ist/sind, die aus Protease, Lipase, Cutinase, Amylase, Carbohydrase, Cellulase, Pektinase, Pektatlyase, Mannanase, Arabinase, Galaktanase, Xylanase, Oxidase, Xanthanase, Laccase und/oder Peroxidase besteht.

4. Detergenszusammensetzung nach einem der Ansprüche 1 bis 3, wobei die Zusammensetzung in Form eines Riegels, einer homogenen Tablette, einer Tablette mit zwei oder mehr Schichten, eines Beutels mit einer oder mehreren Kammer(n), eines regelmäßigen oder kompakten Pulvers, eines Granulats, einer Paste, eines Gels oder einer regelmäßigen, kompakten oder konzentrierten Flüssigkeit vorliegt.

5. Detergenszusammensetzung nach einem der Ansprüche 1 bis 4, wobei die Detergenszusammensetzung eine Waschmittelzusammensetzung ist, vorzugsweise eine flüssige oder feste Waschmittelzusammensetzung.

6. Verwendung einer Detergenszusammensetzung nach einem der Ansprüche 1 bis 5 zum Abbau von Mannan.

7. Verwendung einer Detergenszusammensetzung nach einem der Ansprüche 1 bis 5 in einem Waschprozess.

8. Verfahren zum Entfernen eines Flecks von einer Oberfläche, umfassend das Inkontaktbringen der Oberfläche mit einer Detergenszusammensetzung nach einem der Ansprüche 1 bis 5.

9. Verfahren zum Abbau von Mannan, umfassend das Aufbringen einer Detergenszusammensetzung nach einem der Ansprüche 1 bis 5 auf Mannan, wobei sich das Mannan vorzugsweise auf der Oberfläche eines Textils befindet.

## Revendications

1. Composi on de détergent comprenant au moins une enzyme ayant une séquence d'acides amines ayant au moins 95 % d' iden té de séquences avec la séquence d'acides amines SEQ ID N° 12, l'au moins une enzyme exerçant une ac vité de dégrada on du mannane.

2. Composi on de détergent selon la revendica on 1, dans laquelle l'au moins une enzyme a une séquence d'acides amines ayant au moins 96 %, au moins 97 %, au moins 98 % ou au moins 99 % d'iden té de séquence avec la séquence d'acides amines SEQ ID N° 12.

3. Composi on de détergent selon I'une quelconque des revendica ons 1 à 2, la composi on comprenant en outre au moins une enzyme supplémentaire choisie dans Ie groupe cons tué de la protéase, la lipase, la cu nase, I'amylase, la carbohydrase, la cellulase, la pec nase, la pectatlyase, la mannanase, I'arabinase, la galactanase, la xylanase, I'oxydase, la xanthanase, la laccase et/ou la peroxydase.

4. Composi on de détergent selon I'une quelconque des revendica ons 1 à 3, la composi on étant sous la forme d'une barre, d'un comprime homogène, d'un comprimé comportant au moins deux couches, d'une poche comportant au moins un compar ment, d'une poudre régulière ou compacte, d'une granule, d'une pâte, d'un gel ou d'un liquide régulier, compact ou concentré.

5. Composi on de détergent selon I'une quelconque des revendica ons 1 à 4, la composi on de détergent étant une composi on de détergent de blanchisserie, de préférence une composi on liquide ou solide de détergent de blanchisserie.

6. U lisa on d'une composi on de détergent selon I'une quelconque des revendica ons 1 à 5 des née a la dégrada on du mannane.

7. U lisa on d'une composi on de détergent selon I'une quelconque des revendica ons 1 à 5 dans un processus de blanchisserie.

8. Procédé d'élimina on d'une tache d'une surface, comprenant la mise en contact de la surface avec une composi on de détergent selon I'une quelconque des revendica ons 1 à 5.

9. Procédé de dégrada on du mannane comprenant l'applica on d'une composi on de détergent selon I'une quelconque des revendica ons 1 à 5 sur du mannane, Ie mannane étant de préférence à la surface d'un tex le.
